(19) Europäisches Patentamt
European Patent Office
Office européen des brevets

(11) Veröffentlichungsnummer: **0 235 722**
**B1**

(12)
# EUROPÄISCHE PATENTSCHRIFT

(45) Veröffentlichungstag der Patentschrift: **23.08.89**

(51) Int. Cl.⁴: **C07C 69/618,** C07C 67/14, C07D 317/60, A01N 37/10

(21) Anmeldenummer: **87102557.3**

(22) Anmeldetag: **24.02.87**

(54) **Zimtsäurepropargylester, Verfahren zu ihrer Herstellung und ihre Verwendung zur Schädlingsbekämpfung.**

(30) Priorität: **26.02.86 DE 3606168**

(43) Veröffentlichungstag der Anmeldung:
**09.09.87 Patentblatt 87/37**

(45) Bekanntmachung des Hinweises auf die Patenterteilung:
**23.08.89 Patentblatt 89/34**

(84) Benannte Vertragsstaaten:
**AT BE CH DE FR GB IT LI NL**

(56) Entgegenhaltungen:
**DE-A- 2 210 633**
**DE-A- 2 910 220**
**DE-A- 3 230 775**
**GB-A- 2 002 763**
**GB-A- 2 163 427**

**JOURNAL OF ORGANIC CHEMISTRY, Band 44, Nr. 25, 7. Dezember 1979, Seiten 4524-4527, Columbus, Ohio, US; L.H. KLEMM et al.: "Electroreduction of alpha,beta-unsaturated esters and amides. 3. Polarographic comparison of compounds from saturated and unsaturated alcohols and amines" 000**
**CHEMICAL ABSTRACTS, Band 103, Nr. 1, 8. Juli 1985, Seite 201, Spalte 2, Zusammenfassungsnr. 2085a, Columbus, Ohio, US; G.T. BROOKS et al.: "Inhibitors of juvenile hormone biosynthesis in corpora allata of the**

(73) Patentinhaber: **BASF Aktiengesellschaft, Carl-Bosch-Strasse 38, D-6700 Ludwigshafen(DE)**

(72) Erfinder: **Seppelt, Wolfgang, Dr., Lucas-Cranach-Strasse 8, D-6712 Bobenheim-Roxheim(DE)**
Erfinder: **Neubauer, Hans-Juergen, Dr., B 4,2, D-6800 Mannheim 1(DE)**
Erfinder: **Adolphi, Heinrich, Dr., Kalmitweg 11, D-6703 Limburgerhof(DE)**
Erfinder: **Hofmeister, Peter, Dr., Bernard-Humblot-Strasse 12, D-6730 Neustadt(DE)**

(56) Entgegenhaltungen: (Fortsetzung)
**cockroach Periplaneta americana (L.) in vitro"d alcohols and amines" 000**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es ist bekannt, daß Alkylester von Alkinsäuren oder Alkinylester geeignet sind zur Bekämpfung von Milben (US 4 024 278 bzw. US 3 996 380). Die Wirkung ist jedoch auf Arachniden beschränkt.

In Journal of Organic Chemistry, Band 44, 1979, Z. 4524- 4527, werden polarographische Untersuchungen am unsubstituierten trans-Zimtsäurepropargylester beschrieben.

Es wurde gefunden, daß Zimtsäurepropargylester der Formel

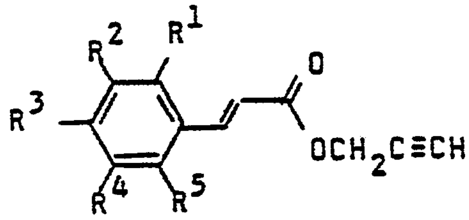

(I),

in der $R^1$, $R^2$, $R^3$, $R^4$ und $R^5$ gleich oder verschieden sind und unabhängig voneinander stehen für H, F, Cl, Br, verzweigtes oder unverzweigtes Alkyl oder Alkenyl mit bis zu 4 Kohlenstoffatomen, Alkoxy oder Alkenyloxy mit bis zu 4 Kohlenstoffatomen, die durch Halogenatome substituiert sein können, wobei $R^2$ und $R^3$ gemeinsam den Molekülanteil $-O-CH_2-O-$ bilden können, und für Phenyl oder Phenoxy, das in 3- und/oder 4-Stellung durch Halogen, Alkyl und Alkoxy mit bis zu 3 Kohlenstoffatomen oder durch den Molekülteil $-O-CH_2-O$ substituiert sein kann, insektizid, akarizid und besonders ovizid bzw. ovolarvizid sehr gut wirksam und bekannten Wirkstoffen ähnlicher Struktur bzw. gleicher Wirkungsrichtung überlegen sind.

Gegenstand der Erfindung sind somit die neuen Zimtsäurepropargylester der Formel I mit der Maßgabe, daß die Reste $R^1$ bis $R^5$ nicht alle gleichzeitig Wasserstoff bedeuten, Verfahren zur Herstellung dieser Verbindungen sowie Mittel, die die oben genannten Zimtsäurepropargylester I als Wirkstoff enthalten.

Die Ester der Formel I können durch Umsetzung entsprechender Zimtsäuren (II) mit Propargylalkohol erhalten werden (vgl. Houben-Weyl, Methoden der organischen Chemie, Band VIII, Seite 516 ff. Georg-Thieme-Verlag, Stuttgart 1952).

Die Umsetzung kann in an sich bekannter Weise durch Zusatz von Katalysatoren wie Schwefelsäure, Halogenwasserstoff, Sulfonsäure, sauren Ionenaustauschern und das Veresterungsgleichgewicht in gewünschtem Sinne verschoben werden, indem man dem Reaktionsgemisch das Wasser oder den Ester I entzieht, z.B. durch azeotrope Destillation oder durch Bindung des Wassers an Schwefel- oder Halogenwasserstoffsäure.

Ebensogut kann man die entsprechenden Säurehalogenide III, in denen Hal(ogen) für Fluor, Chlor und Brom steht, mit Propargylalkohol in Gegenwart eines Säureacceptors umsetzen (vgl. Houben-Weyl, Methoden der organischen Chemie, Band VIII, S. 543 ff., Georg-Thieme-Verlag, Stuttgart 1952).

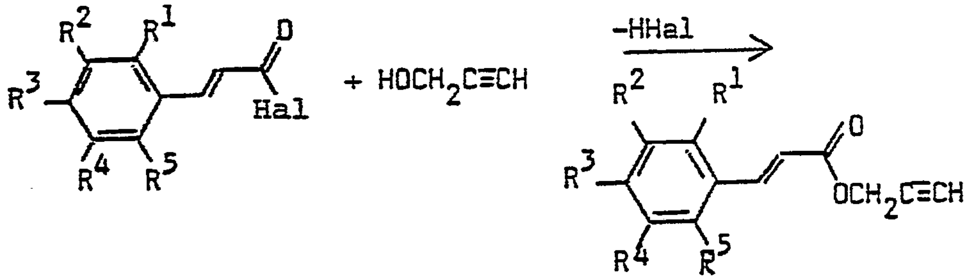


Als Säurebindemittel eignen sich die üblichen basischen Mittel, insbesondere aliphatische, aromatische und heterocylische Amine, z.B. Triethylamin, Dimethylamin, Piperidin, Diazabicytlooctan, Dimethylanilin, Dimethylbenzylamin, Pyridin, Lutidin, Dimethylaminopyridin. Auch Alkalimetallcarbonate, wie Natrium- oder Kaliumcarbonat eignen sich als Säureacceptoren.

Die Umsetzung kann in einem Lösungs- oder Verdünnungsmittel ausgeführt werden. Hierzu sind teilweise die angeführten Säureacceptoren selbst oder beispielsweise folgende Lösungs- oder Verdünnungsmittel oder Gemische derselben geeignet:

Aliphatische und aromatische, gegebenenfalls chlorierte Kohlenwasserstoffe, wie Petrolether, Benzol, Toluol, Xylol, Benzin, Dichlormethan, Chloroform, Tetrachlormethan, 1,2-Dichlormethan, Chlorbenzol, Ether wie Diethyl- und Di-n-butylether, Methyl-tert.-butylether, Tetrahydrofuran, Dioxan; Ketone beispielsweise Aceton, Methylethylketon, Methylisopropylketon; ferner Nitrile wie Acetonitril und Propionitril.

Üblicheweise setzt man die Ausgangsstoffe in stöchiometrischem Verhältnis ein. Ein Überschuß des einen oder anderen kann in Einzelfällen aber durchaus vorteilhaft sein.

Die Umsetzung verläuft gewöhnlich oberhalb 0°C mit ausreichender Geschwindigkeit. Da sie meist unter Wärmeentwicklung verläuft, kann es von Vorteil sein, eine Kühlmöglichkeit vorzusehen.

Die erfindungsgemäßen Zimtsäureproparylester können außerdem noch nach praktisch allen bekannten Verfahren der Estersynthese hergestellt werden, beispielsweise durch Umsetzung von entsprechenden Zimtsäureanhydriden (vgl. Houben-Weyl a. a. O., S. 478) mit Propargylalkohol, durch Umsetzung von entsprechenden Zimtsäuresalzen mit Propargylhalogeniden, durch Umesterungsreaktionen (vgl. Houben-Weyl a. a. O., S. 508 bis 628; C. Ferri, Reaktionen der organischen Synthese, S. 446 ff., Georg-Thieme-Verlag, Stuttgart 1978; S. Patai, The Chemistry of Carboxylic Acids and Esters, S. 505 ff., Interscience Publishers, London 1969), oder beispielsweise aus substituierten aromatischen Aldehyden durch Kondensation mit dem geeignet aktivierten Essigsäureester (s. J. Org. Chem. of USSR (1970), 2309).

Die als Ausgangsmaterial benötigten Zimtsäuren sind entweder bekannt und teilweise handelsüblich, oder lassen sich nach allgemein bekannten chemischen Verfahren herstellen (s. Methodicum Chimicum, Georg Thieme Verlag, Stuttgart 1975; Bd. 5, S. 573 ff. oder z.B. Organic Reaktions, John Wiley and Sons, New York 1967, Vol. 15, p. 204 ff). Die aus ihnen in einigen Fällen hergestellten Zimtsäurechloride wurden z. T. nach Houben- Weyl a. a. O. S. 463 ff. erhalten.

Die Herstellung aller erfindungsgemäßen Verbindungen der Formel (I) ist durch entsprechende Abwandlung des folgenden Beispiels möglich:

Beispiel

12,0 g 2-Methoxy-Zimtsäurechlorid in 30 ml Tetrahydrofuran werden mit 3,75 g Propargylalkohol versetzt und unter Eiskühlung 20 ml Pyridin zugetropft. Man rührt 8 h beim Raumtemperatur, filtriert und engt i.V. ein. Der Rückstand wird in 80 ml Essigester aufgenommen, zweimal mit 10 %iger HCl und zweimal mit Wasser gewaschen. Man trocknet über $Na_2SO_4$ und zieht das Lösungsmittel im Vakuum ab. Der Rückstand wird säulenchromatographisch (Hexan: Essigester 1 : 1) gereinigt. Nach Entfernen des Laufmittels verbleiben 9,9 g des 2-Methoxy-Zimsäurepropargylester als weißes Pulver. FP. 84 bis 87°C

Ausbeute: 9,9 g (75 % d. ber. Menge)

Analyse: Ber. C 72,2H 5.6

Gef. C 72.3H 5.9

$^1H$-NMR ($CDCl_3$, 300 MHz): 8.03 d (1H); 7.50 d (1H); 7.35 t (1H); 6.90 m (2H); 6.58 d (1H); 4.80 sd (2H); 3.90 s (3H); 2.50 st (4H).

Die in der nachstehenden Tabelle mit physikalischen Angaben (Brechungsindex, $n_D$ oder Fp., °C) versehenen Wirkstoffe wurden durch sinngemäße Abwandlung der vorstehenden Vorschrift aus entsprechenden Vorprodukten erhalten; die nicht näher charakterisierten Verbindungen lassen sich auf einem der vorstehend angegebenen Wege erhalten; sie lassen eine vergleichbare biologische Wirkung erwarten.

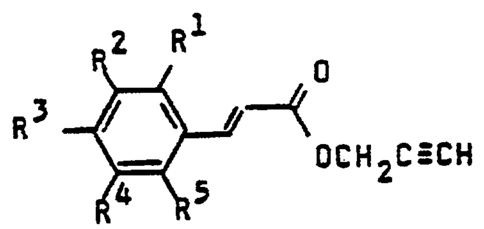

(I)

| Beispiel Nr. | $R^1$ | $R_2$ | $R^3$ | $R^4$ | $R^5$ | $n_D$/Fp. |
|---|---|---|---|---|---|---|
| 2 | F | H | H | H | H | 40°C |
| 3 | Cl | H | H | H | H | 30°C |
| 4 | H | Cl | H | H | H | 43-45°C |
| 5 | H | H | $CH_3$ | H | H | 44-46°C |
| 6 | H | H | i-$C_3H_7$ | H | H | $^{27}$1.5697 |
| 7 | H | H | $OCF_2CF_2H$ | H | H | $^{25}$1.5121 |
| 8 | H | H | F | H | H | 32-34°C |
| 9 | H | H | t-$C_4H_9$ | H | H | $^{21}$1.5618 |
| 10 | H | H | $OCH_3$ | H | H | 45-59°C |
| 11 | H | $OCH_3$ | $OCH_3$ | $OCH_3$ | H | 93°C |
| 12 | Cl | H | Cl | H | H | 92-95°C |
| 13 | H | H | Cl | H | H | 56-58°C |
| 14 | H | H | $OC_2H_5$ | H | H | 47-48°C |
| 15 | H | H | $C_6H_5$ | H | H | 99-100°C |
| 16 | H | $-O-CH_2-O-$ | | H | H | 120-121°C |
| 17 | Cl | Cl | Cl | H | H | 116°C |
| 18 | H | Cl | H | Cl | H | 106°C |
| 19 | H | H | -O 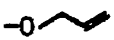 | H | H | $^{24}$1.5986 |
| 20 | H | $CH_3$ | H | H | H | $^{21}$1.5735 |
| 21 | $CH_3$ | $CH_3$ | H | H | H | $^{21}$1.5740 |
| 22 | H | $CH_3$ | Br | H | H | 66-67°C |
| 23 | $OCH_3$ | $OCH_3$ | H | H | H | 76-77°C |
| 24 | H | $OCH_3$ | $OCH_3$ | H | H | 105-106°C |
| 25 | Cl | H | H | H | Cl | 74-75°C |
| 26 | $OCH_3$ | H | H | H | H | 86-87°C |
| 27 | H | F | H | H | H | 31-32°C |
| 28 | H | H | H | H | H | $^{22}$1.5849 |
| 29 | H | H | O-t-$C_4H_9$ | H | H | |
| 30 | H | Br | $OCH_3$ | H | H | 95°C |
| 31 | H | H | $OC_6H_5$ | H | H | 45-47°C |
| 32 | H | H | O- 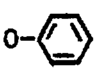 $-OCH_3$ | H | H | 30-40°C |

| Forts. Beispiel Nr. | $R^1$ | $R_2$ | $R^3$ | $R^4$ | $R^5$ | $n_D$/Fp |
|---|---|---|---|---|---|---|
| 33 | H | H | 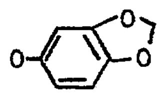 | H | H | 30 |
| 34 | H | H | 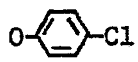 | H | H | 60–70 |
| 35 | H | H | 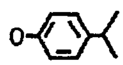 | H | H | 30 |

In den nachstehenden Beispielen zur biologischen Wirkung wurde die aus der US-PS 3 996 380 bekannte Verbindung

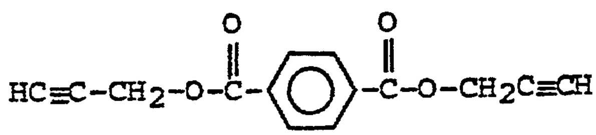

als Vergleichsmittel gewählt.

Prodenia litura (Ägypt. Baumwollwurm), Wirkung auf Eiablagen

Die weiblichen Falter legen ihre Eier in geschlossenen Gruppen auf Pergamentpapier ab. Ausgeschnittene Papierstreifen, die ca. 200 – 300 Eier tragen, werden 2 Tage nach der Ablage ca. 5 Sekunden in die wäßrige Wirkstoffaufbereitung getaucht (Konzentrationsangaben in g Wirkstoff je 100 g Lösung). Anschließend legt man sie in eine Petrischale (Ø 10 cm) auf feuchten Zellstoff.

Die Auswertung erfolgt nach dem Schlüpfen des unbehandelten Kontrollversuches, d.h. nach 5 bis 6 Tagen.

Als wirksam werden die Proben eingestuft, bei denen kein Raupenschlupf erfolgt. Treten vereinzelte Räupchen auf, bewerten wir dieses als Grenze der Wirksamkeit.

Beispiel 2 0,02% ca. 80% Mortalität
6 0,04% ca. 80% Mortalität
28 0,02% ca. 100% Mortalität
Vergleichsmittel 0,1% ca. 45% Mortalität

Dysdercus intermedius (Baumwollwanze), Ovizide Wirkung

Stecketiketten werden am oberen Rand mit Klebestreifenstücken (ca. 0, 8 cm) beklebt.

24 Stunden vor Versuchsbeginn werden die in einem Gefäß gesammelten Eier durch Eintauchen der Etiketten in das Gefäß an dem Klebestreifenrand befestigt.

Die Eier werden für ca. 5 Sekunden in die wäßrige Wirkstoffaufbereitung getaucht und auf Filterpapier abgetropft. Dabei sollen die Eier nicht auf das Papier gelegt werden.

Die zurechtgeschnittenen Etiketten werden in Plastikpaletten gestellt, Klebestreifen nach oben. Eine halbe mit Wasser durchfeuchtete Watterolle wird in den Becher gelegt, um Austrocknung zu vermeiden und die Palette mit einer Glasplatte abgedeckt.

Nach ca. 8 Tagen wird bonitiert (bei der Kontrolle müssen die Larven geschlüpft sein).

In diesem Versuch waren beispielsweise die Verbindungen 2, 5, 8, 13, 14, 21, 27, 28 besser als das Vergleichsmittel.

**Patentansprüche**

1. Zimtsäurepropargylester der Formel (I)

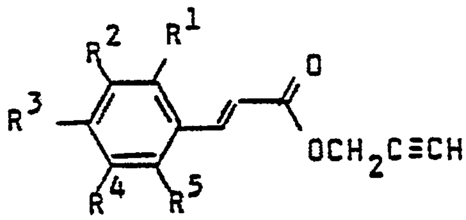

(I),

in der $R^1$, $R^2$, $R^3$, $R^4$ und $R^5$ gleich oder verschieden sind und unabhängig voneinander stehen für H, F, Cl, Br, verzweigtes oder unverzweigtes Alkyl oder Alkenyl mit bis zu 4 Kohlenstoffatomen, Alkoxy oder Alkenyloxy mit bis zu 4 Kohlenstoffatomen, die durch Halogenatome substituiert sein können, wobei $R^2$ und $R^3$ gemeinsam den Molekülteil $-O-CH_2-O-$ bilden können und für Phenyl oder Phenoxy, das in 3- und/oder 4-Stellung durch Halogen, Alkyl und Alkoxy mit bis zu 3 Kohlenstoffatomen oder durch den Molekülteil $-O-CH_2-O-$ substituiert sein kann, mit der Maßgabe, daß die Reste $R^1$ bis $R^5$ nicht alle gleichzeitig Wasserstoff bedeuten.

2. Verfahren zur Herstellung von Zimtsäurepropargylestern der Formel I gemäß Anspruch 1, dadurch gekennzeichnet, daß man eine entsprechende Zimtsäure oder deren Säurehalogenid mit Propargylalkohol, ggf. in Gegenwart eines Säureacceptors umsetzt.

3. Schädlingsbekämpfungsmittel, enthaltend einen Zimtsäurepropargylester der Formel (I)

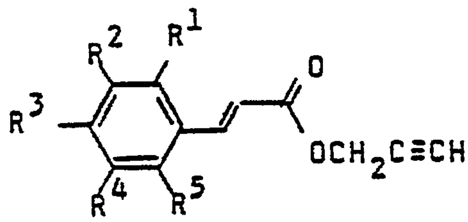

(I),

in der $R^1$, $R^2$, $R^3$, $R^4$ und $R^5$ gleich oder verschieden sind und unabhängig voneinander stehen für H, F, Cl, Br, verzweigtes oder unverzweigtes Alkyl oder Alkenyl mit bis zu 4 Kohlenstoffatomen, Alkoxy oder Alkenyloxy mit bis zu 4 Kohlenstoffatomen, die durch Halogenatome substituiert sein können, wobei $R^2$ und $R^3$ gemeinsam den Molekülteil $-O-CH_2-O-$ bilden können und für Phenyl oder Phenoxy, das in 3- und/oder 4-Stellung durch Halogen, Alkyl und Alkoxy mit bis zu 3 Kohlenstoffatomen oder durch den Molekülteil $-O-CH_2-O-$ substituiert sein kann.

4. Schädlingsbekämpfungsmittel, enthaltend einen festen oder flüssigen Trägerstoff und mindestens einen Zimtsäureester der Formel I gemäß Anspruch 3.

5. Verwendung von Zimtsäureestern der Formel I gemäß Anspruch 3 als Insektizid, Akarizid, insbesondere Ovizid.

6. Verfahren zur Bekämpfung von Schädlingen, dadurch gekennzeichnet, daß man eine wirksame Menge des Zimtsäureesters der Formel I gemäß Anspruch 3 auf Schädlinge bzw. deren Lebensraum einwirken läßt.

**Claims**

1. A propargyl cinnamate of the formula (I)

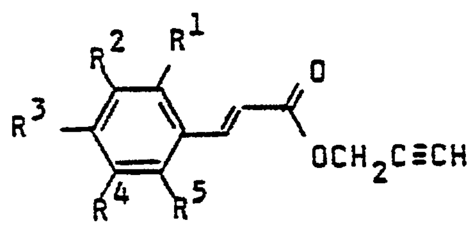

(I),

where $R^1$, $R^2$, $R^3$, $R^4$ and $R^5$ are identical or different and independently of one another are each H, F, Cl, Br, branched or straight-chain alkyl or alkenyl of not more than 4 carbon atoms or alkoxy or alkenyloxy of not more than 4 carbon atoms, which may each be substituted by halogen, and $R^2$ and $R^3$ together may form the moiety $-O-CH_2-O-$, or are each phenyl or phenoxy which may be substituted in the 3- and/or 4-position by halogen, alkyl or alkoxy of not more than 3 carbon atoms or by the moiety $-O-CH_2-O-$, with the proviso that $R^1$ to $R^5$ are not all simultaneously hydrogen.

2. A process for the preparation of a propargyl cinnamate of the formula I as claimed in claim 1, wherein an appropriate cinnamic acid or its acyl halide is reacted with propargyl alcohol, in the presence or absence of an acid acceptor.

3. A pesticide containing a propargyl cinnamate of the formula (I)

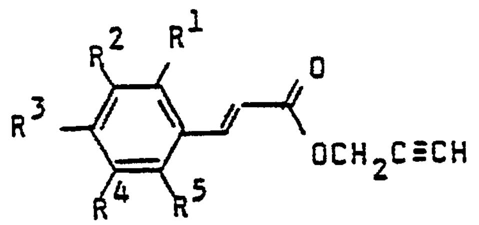

(I),

where $R^1$, $R^2$, $R^3$, $R^4$ and $R^5$ are identical or different and independently of one another are each H, F, Cl, Br, branched or straight-chain alkyl or alkenyl of not more than 4 carbon atoms or alkoxy or alkenyloxy of not more than 4 carbon atoms, which may each be substituted by halogen, and $R^2$ and $R^3$ together may form the moiety $-O-CH_2-O-$, or are each phenyl or phenoxy which may be substituted in the 3- and/or 4-position by halogen, alkyl or alkoxy of not more than 3 carbon atoms or by the moiety $-O-CH_2-O-$.

4. A pesticide containing a solid or liquid carrier and one or more cinnamates of the formula I as claimed in claim 3.

5. Use of a cinnamate of the formula I as claimed in claim 3 as an insecticide, acaricide or, in particular, ovicide.

6. A method of controlling pests, wherein an effective amount of the cinnamate of the formula I as claimed in claim 3 is allowed to act on pests or their habitat.

**Revendications**

1. Ester propargylique d'acide cinnamique de formule (I)

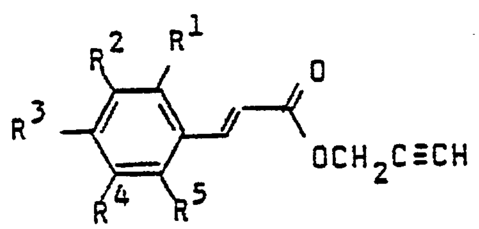

(I),

dans laquelle $R^1$, $R^2$, $R^3$, $R^4$ et $R^5$ sont identiques ou différents et indépendamment l'un de l'autre sont mis pour H, F, Cl, Br, alkyle ou alcényle ramifié ou non ramifié, ayant jusqu'à 4 atomes de carbone, alcoxy ou alcényloxy ayant jusqu'à 4 atomes de carbone, qui peuvent être substitués par des atomes d'halogène, $R^2$ et $R^3$ pouvant, ensemble, former la partie de molécule $-O-CH_2-O-$ et pour phényle ou phénoxy, qui peut être substitué, en position 3 et/ou 4 par halogène, alkyle ou alcoxy ayant jusqu'à 3 atomes de carbone ou pour une partie de molécule $-O-CH_2-O$, sous réserve que les restes $R^1$ à $R^5$ ne représentent pas tous simultanément de l'hydrogène.

2. Procédé de préparation d'ester propargylique d'acide cinnamique de formule (I) selon la revendication 1, caractérisé par le fait que l'on fait réagir un acide cinnamique correspondant ou son halogénure d'acide avec de l'alcool propargylique, éventuellement en présence d'un accepteur d'acide.

3. Produit antiparasitaire, contenant un ester propargylique d'acide cinnamique de formule (I) dans lequel $R^1$, $R^2$, $R^3$, $R^4$ et $R^5$ sont identiques on différents et indépendamment l'un de l'autre sont mis pour H, F, Cl, Br, alkyle on alcényle ramifié ou non ramifié, ayant jusqu'à 4 atomes de carbone, alcoxy ou alcényloxy ayant jusqu'à 4 atomes de carbone, qui peuvent être substitués par des atomes d'halogène, $R^2$ et $R^3$ pouvant, ensemble, former la partie de molécule $-O-CH_2-O-$ et pour phényle et phénoxy, qui peut être substitué, en position 3 et/ou, 4 par halogène, alkyle ou alcoxy ayant jusqu'à 3 atomes de carbone ou par une partie de molécule $-O-CH_2-O$.

4. Agent antiparasitaire, contenant un support solide ou liquide et au moins un ester d'acide cinnamique de formule I selon la revendication 3.

5. Utilisation d'esters d'acide cinnamique de formule I selon la revendication 3, comme insecticide, acaracide, en particulier ovicide.

6. Procédé pour lutter contre les parasites, caractérisé par le fait que l'on fait agir sur les parasites ou leur biotope une quantité efficace d'ester d'acide ciannamique de formule I selon la revendication 3.